(19) Europäisches Patentamt
European Patent Office
Office européen des brevets

(11) Veröffentlichungsnummer: **0 007 010**
**A1**

(12) # EUROPÄISCHE PATENTANMELDUNG

(21) Anmeldenummer: 79101917.7

(22) Anmeldetag: 13.06.79

(51) Int. Cl.$^3$: C 07 D 249/08
A 01 N 43/64

(30) Priorität: 19.06.78 DE 2826760

(43) Veröffentlichungstag der Anmeldung: 23.01.80 Patentblatt 80/2

(84) Benannte Vertragsstaaten: AT BE CH DE FR GB IT NL SE

(71) Anmelder: HOECHST AKTIENGESELLSCHAFT
Zentrale Patentabteilung Postfach 80 03 20
D-6230 Frankfurt/Main 80(DE)

(72) Erfinder: Heubach, Günther, Dr.
Luisenstrasse 15
D-6233 Kelkheim (Taunus)(DE)

(72) Erfinder: Sachse, Burkhard, Dr.
Parkstrasse 18
D-6233 Kelkheim (Taunus)(DE)

(72) Erfinder: Bürstell, Helmut, Dr.
Am Dachsberg 89
D-6000 Frankfurt am Main(DE)

(54) **1,2,4-Triazolderivate, Verfahren zu deren Herstellung, deren Verwendung als Schädlingsbekämpfungsmittel und Pflanzenwuchsregulatoren und diese Derivate enthaltende Schädlingsbekämpfungsmittel.**

(57) Gegenstand der Erfindung sind neue Verbindungen der Formel I,

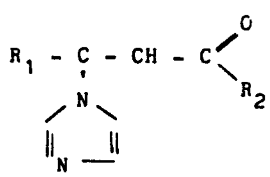

in der
$R_1$ Methyl oder Phenyl, das gegebenenfalls durch Halogenatome, $(C_1$-$C_4)$-Alkylreste oder $(C_1$-$C_4)$-Alkoxyreste substituiert ist, und
$R_2$ eine $R_3O$-Gruppe, in der $R_3$ Alkyl das gegebenenfalls zusätzlich durch Halogen, $(C_1$-$C_4)$-Alkoxyreste oder Alkoxycarbonylreste, substituiert ist, Alkinyl Cycloalkyl, das gegebenenfalls zusätzlich durch $(C_1$-$C_4)$-Alkylreste substituiert ist, Phenyl, das gegebenenfalls durch Halogenatome und/oder die $F_3C$-Gruppe substituiert ist, oder
$R_2$ eine $R_4$-N-Gruppe,
$R_5$

in der $R_4$ Wasserstoff oder $(C_1$-$C_4)$-Alkyl, das gegebenenfalls durch Alkoxycarbonyl substituiert ist, und
$R_5$ $(C_1$-$C_4)$-Alkyl oder Phenyl, das gegebenenfalls durch Halogenatome, $F_3C$-Reste, $(C_1$-$C_4)$-Alkyl-oder $(C_1$-$C_4)$-Alkoxyreste oder durch Phenoxy oder Halogenphenoxy substituiert ist, bedeutet, sowie ein Verfahren zu deren Herstellung durch Umsetzung von 1,2,4-Triazol mit einem entsprechenden Halogenid, sowie ihre Verwendung zur Schädlingsbekämpfung und als Pflanzenwachstumsregulatoren. Die Herstellung des als Ausgangsstoff verwendeten Halogenids erfolgt aus dem zugehörigen Säurehalogenid.

EP 0 007 010 A1

HOECHST AKTIENGESELLSCHAFT HOE 78/F 122 Dr. GM/ss

**Verfahren zur Herstellung von Derivaten des 1,2,4-Triazols und deren Verwendung als Schädlingsbekämpfungsmittel und Pflanzenwuchsregulatoren**

Gegenstand vorliegender Erfindung sind neue Derivate des 1,2,4-Triazols, ihre Herstellung und Verwendung als Schädlingsbekämpfungsmittel im Pflanzenschutz sowie als Pflanzenwachstumsregulatoren.

Die erfindungsgemäßen Verbindungen besitzen die allgemeine Formel I,

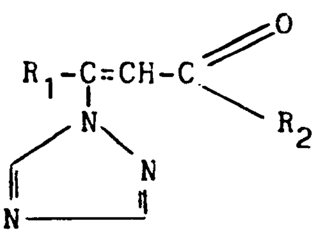

(I)

in der $R_1$ und $R_2$ folgende Bedeutungen haben:

$R_1$ Methyl oder Phenyl, das gegebenenfalls durch Halogenatome, $(C_1-C_4)$-Alkylreste, vorzugsweise $(C_1-C_2)$-Alkylreste, oder durch $(C_1-C_4)$-Alkoxyreste, vorzugsweise $(C_1-C_2)$-Alkoxyreste substituiert ist,

$R_2$ eine $R_3O$-Gruppe, in der $R_3$ Alkyl, vorzugsweise mit bis zu 12, insbesondere mit bis zu 8 C-Atomen, das gegebenenfalls zusätzlich durch Halogen, vorzugsweise Chlor oder Brom, durch $(C_1-C_4)$-Alkoxyreste oder durch Alkoxycarbonylreste, vorzugsweise mit bis zu 8, insbesondere mit bis zu 5 C-Atomen substituiert ist, Alkinyl, vorzugsweise mit bis zu 3 C-Atomen, Cycloalkyl, vorzugsweise mit bis zu 6 C-Atomen, das gegebenenfalls zusätzlich durch $(C_1-C_4)$-Alkylreste substituiert ist, Phenyl, das gegebenenfalls durch Halogenatome und/ oder die $F_3C$-Gruppe substituiert ist, oder

$R_2$ eine $R_4$-N-Gruppe,
$R_5$

in der $R_4$ Wasserstoff oder $(C_1-C_4)$-Alkyl, das gegebenenfalls durch Alkoxycarbonyl, vorzugsweise mit bis zu 8, insbesondere mit bis zu 5 C-Atomen substituiert ist und $R_5$ $(C_1-C_4)$-Alkyl oder Phenyl, das gege-

benenfalls durch Halogenatome, $F_3C$-Reste, $(C_1-C_4)$-Alkyl- oder $(C_1-C_4)$-Alkoxyreste oder durch Phenoxy oder Halogenphenoxy substituiert ist, bedeutet.

Die Verbindungen der Formel I fallen im allgemeinen als cis/trans-Isomerengemische an und können durch an sich bekannte Verfahren in die einzelnen Isomeren getrennt werden.

Die Triazolderivate der Formel I können dadurch hergestellt werden, daß man Verbindungen der allgemeinen Formel II,

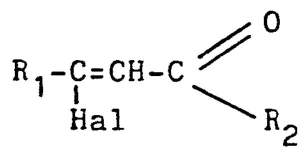

(II)

in der $R_1$ und $R_2$ die Bedeutungen wie in Formel I haben und Hal Chlor oder Brom, vorzugsweise Chlor bedeutet, bei höheren Temperaturen, vorzugsweise bei 70 bis 140°C und insbesondere bei 80 bis 110°C mit 1,2,4-Triazol in Gegenwart eines Halogenwasserstoff bindenden Mittels umsetzt. Die Umsetzungen führt man zweckmäßigerweise in organischen Lösungsmitteln, wie z.B. Aceton, Acetonitril, Dimethylformamid oder höhersiedenden Kohlenwasserstoffen, wie z.B. Xylol durch, was vorteilhaft sein kann. Man kann zum Abfangen der bei der Umsetzung gebildeten Halogenwasserstoffsäure vorteilhaft einen mindestens molaren Überschuß an Triazol oder auch entsprechende Mengen anorganischer oder tertiärer organischer Basen einsetzen, wie z.B. $K_2CO_3$, $Na_2CO_3$, Triäthylamin, Pyridin oder N,N-Dimethylanilin.

Die zum größten Teil ebenfalls neuen Vorprodukte der Formel II können in an sich bekannter Weise aus den Säurechloriden der Formel III

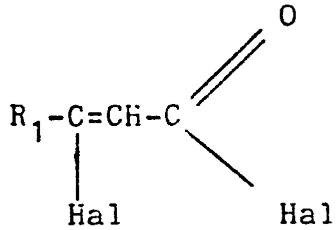

(III)

durch Umsetzung mit Alkoholen der allgemeinen Form $R_2OH$ bzw. Aminen der allgemeinen Formel

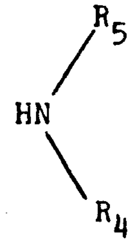
,

wobei $R_1$, $R_2$, $R_4$, $R_5$ und Hal die Bedeutungen wie in Formeln I und II haben, erhalten werden.

Die Carbonsäurechloride der Formel III lassen sich nach literaturbekannten Verfahren, z.B. durch Umsetzung der ß-Ketoester $R_1$-CO-$CH_2$-COOR mit Chlorierungsmitteln herstellen /¯JACS 77, 1137(1955); J.Chem. Soc. 99, 1621 (1911)_7.

Die beanspruchten Verbindungen der allgemeinen Formel I zeichnen sich durch eine sehr gute fungizide Wirkung aus. Mit ihrer Hilfe lassen sich bereits in das pflanzliche Gewebe eingedrungene pilzliche Krankheitserreger kurativ bekämpfen. Dies ist besonders wichtig und vorteilhaft bei solchen Pilzkrankheiten, die nach eingetretener Infektion mit den sonst üblichen Fungiziden nicht mehr bekämpft werden können. Das Wirkungsspektrum der beanspruchten Verbindungen erfaßt z.B. neben Phytophthora infestans, Plasmopara viticola, Venturia inaequalis, Cercospora betae und Tilletia tritici vor allem Echte Mehltauarten im Obst-, Gemüse-, Getreide-, Wein- und Zierpflanzenbau und teilweise auch Rostpilze. Besonders hervorzuheben ist die ausgezeichnete Wirkung der Verbindungen gegen Mehltauarten, die gegen Benzimidazolderivate (z.B. Benomyl, Carbendazim) resistent sind.

Die beanspruchten Verbindungen der Formel I eignen sich auch für den Einsatz im technischen Bereich, beispielsweise in Holzschutzmitteln, auf dem Anstrichfarbensektor, als Konservierungsmittel z.B. in Kühlschmiermitteln für die Metallbearbeitung. Darüber hinaus zeigen einige der

Verbindungen eine gute Wirkung gegen phytopathogene Bakterienarten, deren Bekämpfung zunehmende Bedeutung in gärtnerischen und landwirtschaftlichen Kulturen gewinnt.

Die beanspruchten Verbindungen der Formel I zeigten außerdem in biologischen Versuchen sehr gute pflanzenwachstumsregulierende Eigenschaften bei einer Reihe von wirtschaftlich wichtigen Pflanzenarten. Insbesondere hervorzuheben ist neben der allgemeinen wachstumsregulatorischen Wirksamkeit die Wachstumshemmung in Getreide.

Es ist bereits bekannt, daß zur Regulierung des Wachstums von höheren Pflanzen Bernsteinsäure-2,2-dimethylhydrazid, 2-Chloräthyl-trimethylammoniumchlorid und Maleinsäurehydrazid verwendet werden können. Die Wirkung dieser Verbindungen ist jedoch nicht immer befriedigend, da bei ihrer Anwendung z.B. bei höheren Konzentrationen teilweise phytotoxische Schäden auftreten können, bestimmte wichtige Kulturpflanzen nicht erfaßt werden oder Ertragseinbußen auftreten. Dagegen konnten überraschenderweise mit den beanspruchten Verbindungen der Formel I bei sehr guter regulatorischer Wirksamkeit keine phytotoxischen Schäden beobachtet werden.

Für die Anwendung im Plfanzenschutz können die Verbindungen der Formel I in üblicher Weise als Stäube, Spritzpulver, Beizmittel, Dispersionen, Lösungen oder Emulsionskonzentrate formuliert werden. Der Gehalt an Wirkstoff der Formel I in solchen Formulierungen liegt im allgemeinen zwischen 2 und 95 Gew.-%, vorzugsweise bei 10-90 Gew.-%. Daneben enthalten die genannten Wirkstoff-Formulierungen gegebenenfalls die jeweils üblichen Haft-, Netz-, Dispergier-, Emulgier-, Penetrations-, Lösungsmittel-, Füll- und Trägerstoffe.

Die Erfindung wird durch die nachfolgenden Beispiele erläutert.

## A. HERSTELLUNGSBEISPIELE

Beispiel 1:

3-(1,2,4-Triazol-1-yl)-zimtsäure-N-isopropyl-2',6'-dimethylanilid

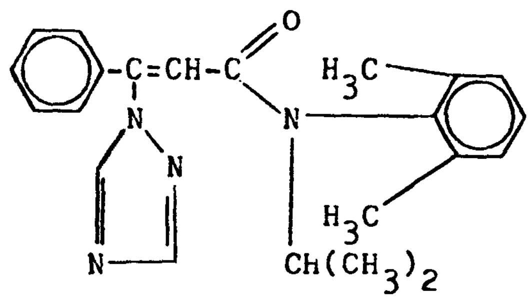

0,07 Mol 3-Chlor-zimtsäure-N-isopropyl-2',6'-dimethylanilid (22,9 g) wurden in 90 ml Dimethylformamid gelöst, mit 5,8 g 1,2,4-Triazol (0,084 Mol) und 11,5 g wasserfreiem Kaliumcarbonat versetzt und unter Rühren 6 Stunden bei 100°C gehalten. Nach Abkühlen des Reaktionsgemisches und Eingießen in Eiswasser wurde mit Methylenchlorid extrahiert und die organische Phase mit Wasser gewaschen. Nach Entfernen des Methylenchlorids unter Vakuum hinterblieben 23,6 g bernsteinfarbenes semikristallines Produkt, das gemäß der Dünnschichtchromatographie zu etwa gleichen Teilen aus 3-(1,2,4-Triazol-1-yl)-zimtsäure-N-isopropyl-2, 6'-dimethylanilid cis-trans Isomeren bestand.

Ausbeute: 23,6 g = 93,5 % d.Th.

Dünnschichtchromatogramm: $R_f$ 0,4 und 0,5
(DC-Fertigplatten, Kieselgel 60 $F_{254}$-Merck)
Laufmittel: Äthylacetat

Analyse: $C_{22}H_{24}N_4O$ MG 360,4

| | | | | | | |
|---|---|---|---|---|---|---|
| ber.: | C | 73,30 %; | H | 6,71 %; | N | 15,54 % |
| gef.: | C | 73,2 %; | H | 6,7 %; | N | 15,5 % |

Umkristallisieren einer Probe aus Toluol/Benzin ergab farblose Kristalle mit einem Schmelzpunkt von 160°-161°C und korrekten Analysenwerten. NMR-Spektrum und Dünnschichtchromatogramm zeigen eine starke Anreicherung eines der beiden Isomeren.

**Beispiel 2:**

**3-(1,2,4-Triazol-1-yl)-4'-chlor-zimtsäureisobutylester**

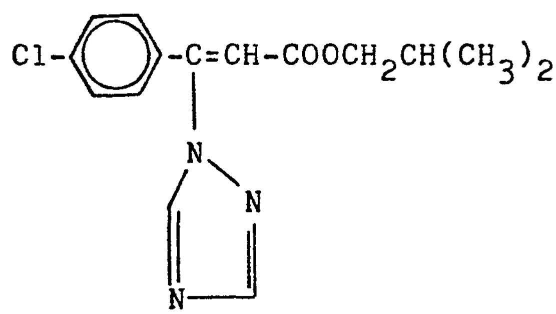

0,07 Mol 3-Chlor-4'-chlor-zimtsäureisobutylester (19,1 g) wurden analog Beispiel 1 mit 1,2,4-Triazol umgesetzt. Nach Aufarbeitung erhält man einen honigfarbenen, zähen Sirup, der im Dünnschichtchromatogramm (Laufmittel: Toluol/Äthylacetat = 2/1) mit $R_f$-Werten von 0,38 und 0,45 das Vorliegen eines 3-(1,2,4-Triazol-1-yl)-4'-chlor-zimtsäureisobutylester cis/trans-Gemisches anzeigt.

Ausbeute: 19,5 g = 91 % d. Th.

Analyse: $C_{15}H_{16}ClN_3O_2$ MG 305,7

| | | | | |
|---|---|---|---|---|
| ber.: | C | 58,92 %; | H 5,27 %; | N 13,74 % |
| gef.: | C | 58,6 %; | H 5,0 %; | N 13,5 % |

**Beispiel 3:**

**3-(1,2,4-Triazol-1-yl)-crotonsäureisobutylester**

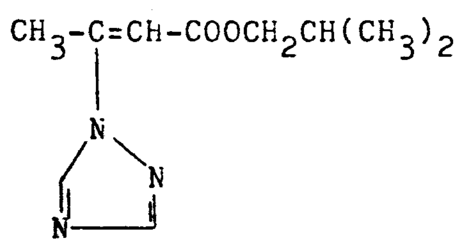

0,1 Mol 3-Chlor-crotonsäureisobutylester (17,7 g) wurden in 100 ml Acetonitril mit 0,12 g 1,2,4-Triazol (8,3 g) und 0,12 Mol wasserfreiem Kaliumcarbonat (16,5 g) 5 Stunden unter Rückfluß gehalten. Nach Entfernen des Lösungsmittels unter Vakuum wurde das Reaktionsgemisch abgekühlt, mit Wasser versetzt und mit Methylenchlorid ausgeschüttelt. Nach Abdestillieren des Methylenchlorids unter Vakuum hinterblieb der 3-(1,2,4-Triazol-1-yl)-crotonsäureisobutylester als ein schwach gelb gefärbtes Öl.

Ausbeute: 18,2 g = 87 % d.Th.

Dünnschichtchromatogramm (Toluol/Äthylacetat = 2 : 1): $R_f$ 0,2

Analyse: $C_{10}H_{15}N_3O_2$ MG 209,2

| | | | |
|---|---|---|---|
| ber.: | C 57,40 %; | H 7,23 %; | N 20,08 % |
| gef.: | C 57,1 %; | H 7,3 %; | N 19,8 % |

<u>Beispiele 4-90:</u>

In analoger Weise wie in Beispiel 3 beschrieben wurden die Beispiele 4 bis 6 ausgeführt, während die Beispiele 7 bis 90 analog dem Beispiel 1 ausgeführt wurden. In der Tabelle 1 sind die Reste $R_1$ und $R_2$ in Formel I der nach den Beispielen 4 bis 90 aus den entsprechenden Verbindungen der Formel II hergestellten Verbindungen und deren Schmelzpunkte bzw. deren Brechungsindex aufgeführt.

Tabelle 1

Formel I:

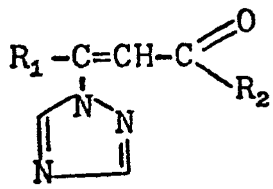

| Beispiel Nr. | $R_1$ | $R_2$ | Schmp. (°C) |
|---|---|---|---|
| 4 | $-CH_3$ | 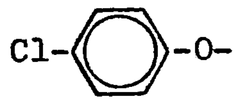 | 106-107 |
| 5 | $-CH_3$ | $(CH_3)_2CHO-$ | 42-44<br>0,3/105 |
| 6 | $-CH_3$ | $CH_3(CH_2)_3O-$ | Oel |
| 7 | 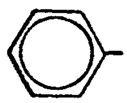 | $CH_3O-$ | 74-88 |
| 8 | 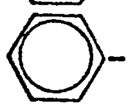 | $C_2H_5O-$ | Oel |
| 9 | 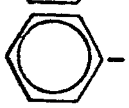 | $(CH_3)_2CHO-$ | 0,3/147 |
| 10 | 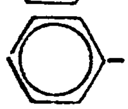 | $CH_3CH_2CH_2O-$ | Oel |
| 11 | 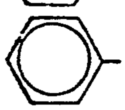 | $(CH_3)_2CHCH_2O-$ | Oel |
| 12 | 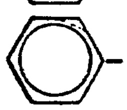 | $CH_3(CH_2)_3O-$ | Oel |
| 13 | 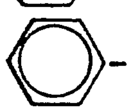 | $(CH_3)_3CO-$ | semi-kristallin |
| 14 | 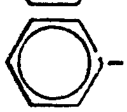 | 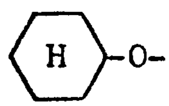 | Oel |
| 15 | 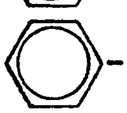 | $H_5C_2OOC-CH(CH_3)-O-$ | Oel |
| 16 | 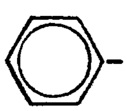 | $CH{\equiv}C-CH_2O-$ | Oel |
| 17 | 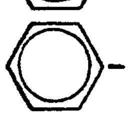 | 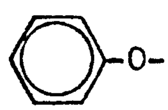 | 99-115 |
| 18 | 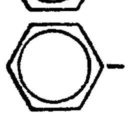 | 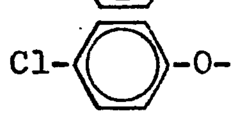 | 108-121<br>(138-140)$^{+)}$ |

| Beispiel Nr. | $R_1$ | $R_2$ | Schmp. (°C) |
|---|---|---|---|
| 19 | 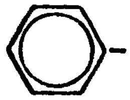 | $-N(C_2H_5)_2$ | Oel |
| 20 | 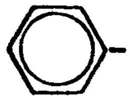 | $-N(CH_2CH_2CH_3)_2$ | Oel |
| 21 | 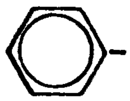 | $-N[CH(CH_3)_2]_2$ | 150-151 |
| 22 | 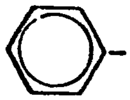 | $-\underset{H}{N}CH(CH_3)_2$ | 160-162 |
| 23 | 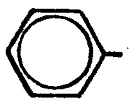 | 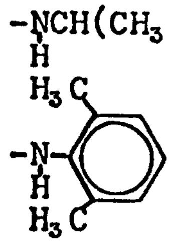 | 243-255 (265-266)+) |
| 24 | 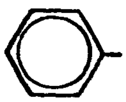 | 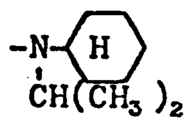 | Sirup |
| 25 | 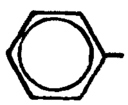 | 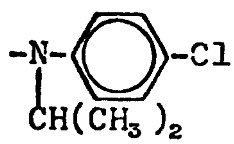 | Oel |
| 26 | 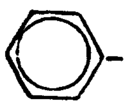 | 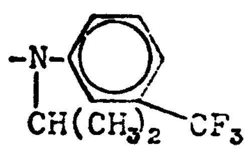 | Oel |
| 27 | 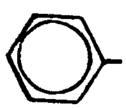 | 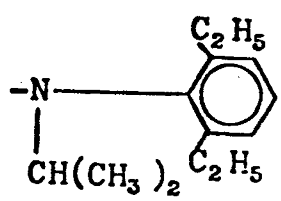 | Sirup |
| 28 | 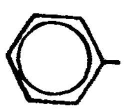 | 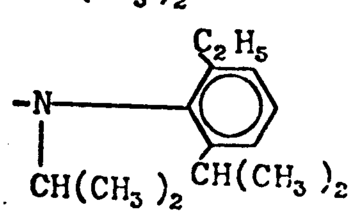 | Sirup |
| 29 | 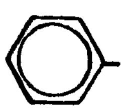 | 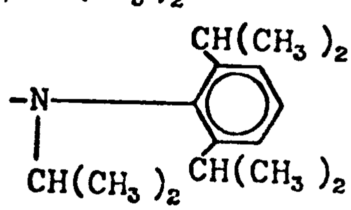 | 113-119 |

| Beispiel Nr. | $R_1$ | $R_2$ | Schmp.(°C) |
|---|---|---|---|
| 30 | 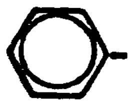 | 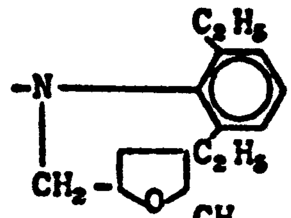 | Sirup |
| 31 | 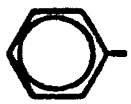 | 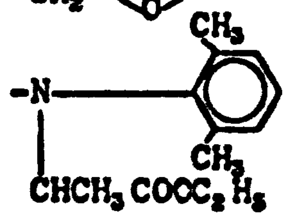 | semi-kristallin |
| 32 | 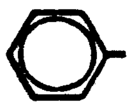 | 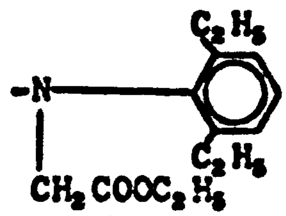 | Sirup |
| 33 | 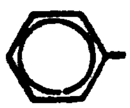 | 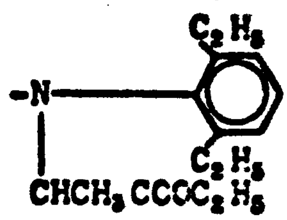 | Sirup |
| 34 | Cl- 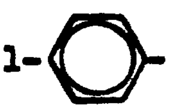 | $-O(CH_2)_2CH_3$ | semi-kristallin |
| 35 | Cl- 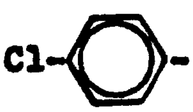 | $-OCH(CH_3)_2$ | Oel |
| 36 | Cl- 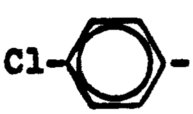 | $-O(CH_2)_3CH_3$ | semi-kristallin (100-104)+) |
| 37 | Cl- 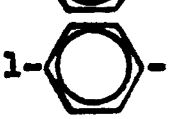 | $-O-C(CH_3)_3$ | Sirup |
| 38 | Cl- 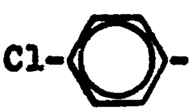 | 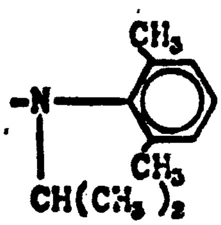 | 104-122 (122-133)+) |

| Beispiel Nr. | $R_1$ | $R_2$ | Schmp. (°C) |
|---|---|---|---|
| 39 | 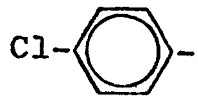 | 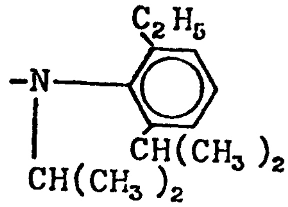 | Sirup |
| 40 | 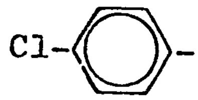 | 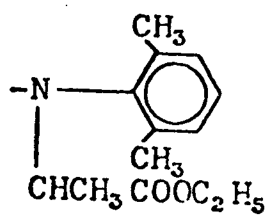 | Sirup |
| 41 | 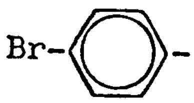 | 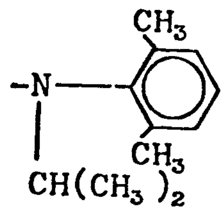 | semi-kristallin, (163-164)+) |
| 42 | 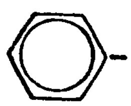 | 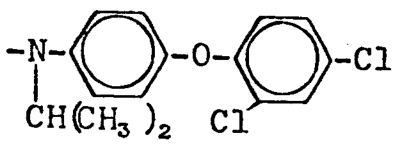 | Sirup |
| 43 | 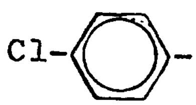 | 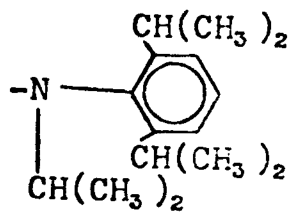 | 129-157 |
| 44 | 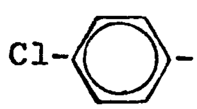 | 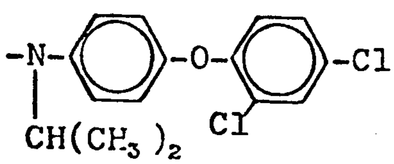 | Sirup |
| 45 | 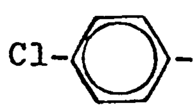 | $-OCH_3$ | 117-122 |
| 46 | 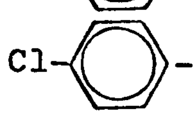 | $-OC_2H_5$ | semi-kristallin |
| 47 | 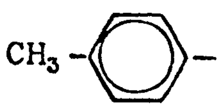 | 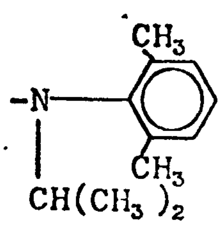 | 105-135 |

| Beispiel Nr. | $R_1$ | $R_2$ | Schmp.(°C) |
|---|---|---|---|
| 48 | $CH_3$- 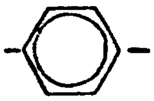 | $-O(CH_2)_3CH_3$ | Sirup |
| 49 | Cl- 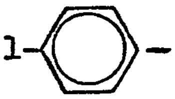 | -O- 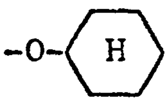 | Sirup |
| 50 | Cl- 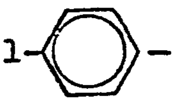 | 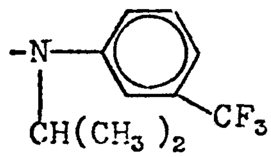 | Sirup |
| 51 | Cl- 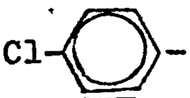 | $-N(CH_2CH_2CH_3)_2$ | 117-120 |
| 52 | $H_3CO$- 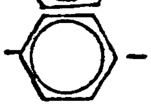 | $-O-(CH_2)_3CH_3$ | Oel |
| 53 | $H_3CO$- 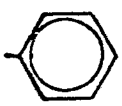 | 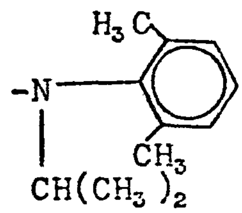 | Sirup |
| 54 | Cl- 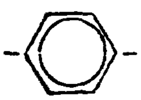 | $-O-(CH_2)_4-CH_3$ | Sirup |
| 55 | Cl- 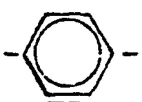 | $-O-(CH_2)_5-CH_3$ | semi-kristallin |
| 56 | Cl- 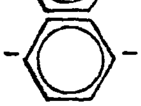 | $-O-(CH_2)_2-CH(CH_3)-CH_3$ | Sirup |

+) Schmelzpunkt der aus Toluol/Hexan umkristallisierten Proben

| Beisp. Nr. | $R_1$ | $R_2$ | $n_D^{30}$ (Brechungsindex) [bei zähen, sirupartigen Produkten nicht gemessen] |
|---|---|---|---|
| 57 | 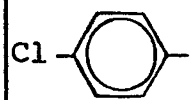 | 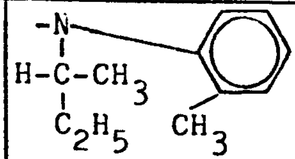 | 1,5779 |
| 58 | 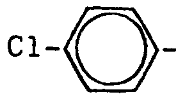 | 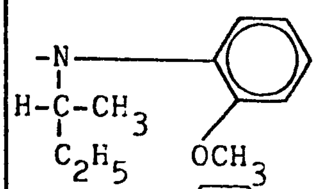 | 1,5798 |
| 59 | 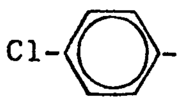 | 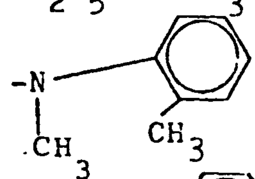 | 1,5926 |
| 60 | 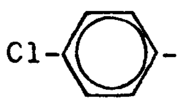 | 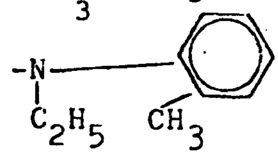 | 1,5859 |
| 61 | 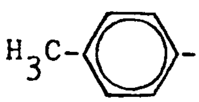 | 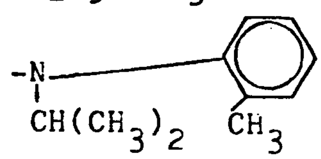 | 1,5688 |
| 62 | 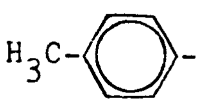 | 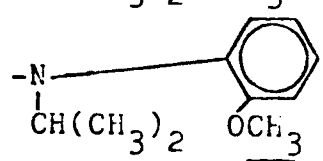 | 1,5755 |
| 63 | 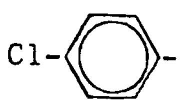 | 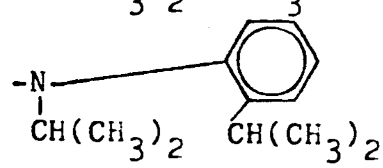 | 1,5768 |
| 64 | 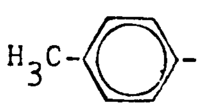 | 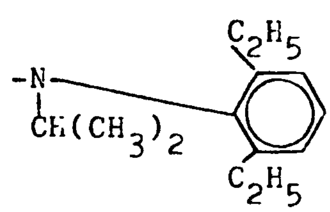 | 1,5843 |
| 65 | 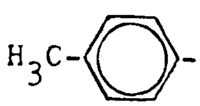 | 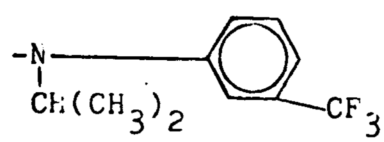 | 1,5532 |
| 66 | 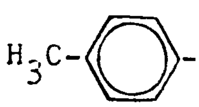 | $-OCH(CH_3)_2$ | 1,5587 |
| 67 | 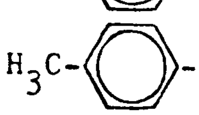 | 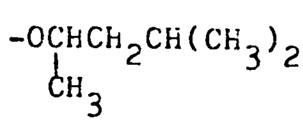 | 1,5501 |

| Beisp. Nr. | $R_1$ | $R_2$ | $n_D^{30}$ (Brechungsindex) /bei zähen, sirupartigen Produkten nicht gemessen/ |
|---|---|---|---|
| 68 | 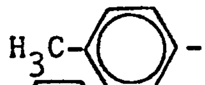 | $-O(CH_2)_2CH_3$ | 1,5602 |
| 69 | 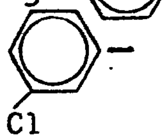 | $-OCH(CH_3)_2$ | 1,5583 |
| 70 | 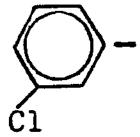 | $-OCH_2CH(CH_3)_2$ | 1,5372 |
| 71 | 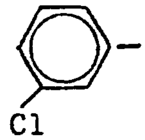 | $-O(CH_2)_2CH_3$ | 1,5682 |
| 72 | 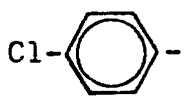 | 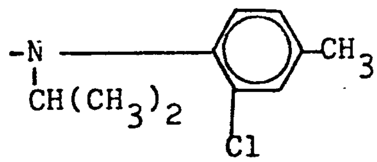 | 1,5812 |
| 73 | 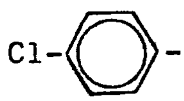 | 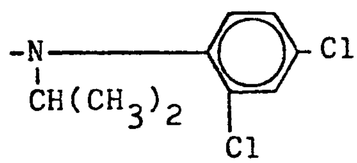 | 1,5863 |
| 74 | 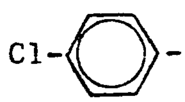 | 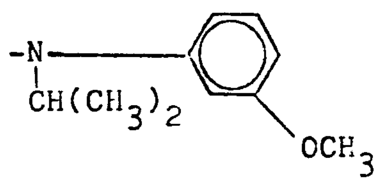 | Sirup |
| 75 | 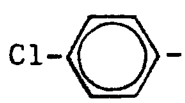 | 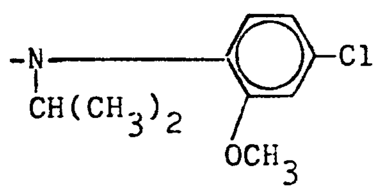 | Sirup |
| 76 | 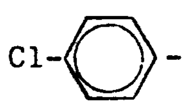 | 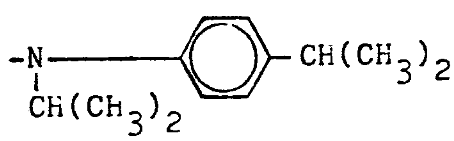 | Sirup |
| 77 | 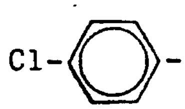 | 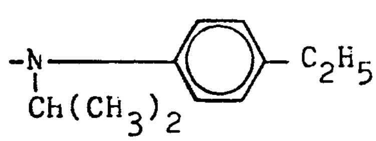 | semikristallin |
| 78 | 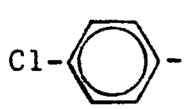 | 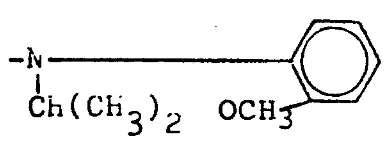 | semikristallin |

| Beisp. | $R_1$ | $R_2$ | $n_D^{30}$ (Brechungsindex) /Bei zähen, sirupartigen Produkten nicht gemessen/ |
|---|---|---|---|
| 79 | 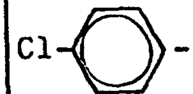 | 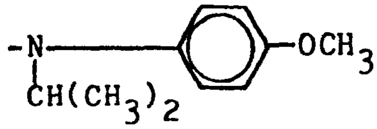 | Sirup |
| 80 | 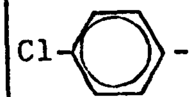 | 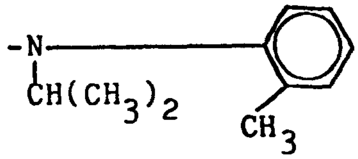 | 1,5328 |
| 81 | 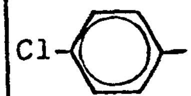 | 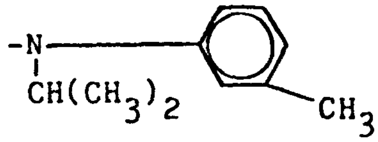 | Sirup |
| 82 | 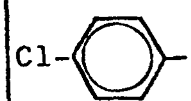 | 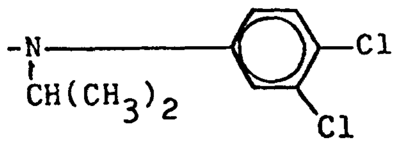 | semikristallin |
| 83 | 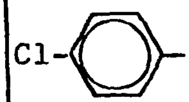 | 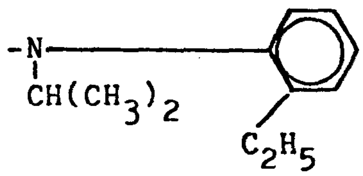 | Sirup |
| 84 | 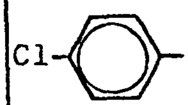 | $-OCH_2CH(CH_2)_3CH_3$ with $C_2H_5$ on CH | Sirup |
| 85 | 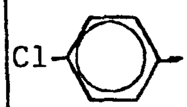 | $-OCHCH_2CH(CH_3)_2$ with $CH_3$ on first CH | Sirup |
| 86 | 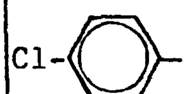 | $O-(CH_2)_7CH_3$ | semikrstallin |
| 87 | 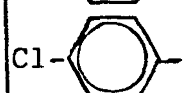 | $-O(CH_2)_2OC_2H_5$ | Sirup |
| 88 | 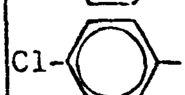 | $-O(CH_2)_3Cl$ | Sirup |
| 89 | 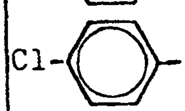 | 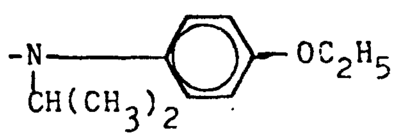 | 1,5789 |
| 90 | 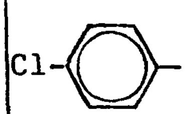 | 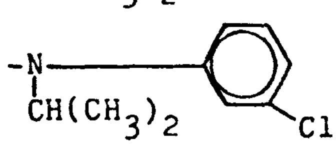 | semikristallin |

## B. FORMULIERUNGSBESPIELE

### Beispiel A:

Ein Stäubemittel wird erhalten, indem man

| | | |
|---|---|---|
| | 10 Gewichtsteile | Wirkstoff |
| und | 90 Gewichtsteile | Talkum als Inertstoff |

mischt und in einer Schlagmühle zerkleinert.

### Beispiel B:

Ein in Wasser leicht dispergierbares benetzbares Pulver wird erhalten, indem man

| | | |
|---|---|---|
| | 25 Gewichtsteile | Wirkstoff |
| | 64 Gewichtsteile | kaolinhaltiger Quarz als Inertstoff |
| | 10 Gewichtsteile | ligninsulfonsaures Kalium |
| und | 1 Gewichtsteil | oleoylmethyltaurinsaures Natrium als Netz- und Dispergiermittel |

mischt und in einer Stiftmühle mahlt.

### Beispiel C:

Ein in Wasser leicht dispergierbares Dispersionskonzentrat wird erhalten, indem man

| | | |
|---|---|---|
| | 20 Gewichtsteile | Wirkstoff mit |
| | 6 Gewichtsteilen | Nonylphenolpolyglykoläther (10 AeO) |
| | 3 Gewichtsteilen | Isotridecanolpolyglykoläther (8 AeO) |
| und | 71 Gewichtsteilen | paraffinischem Mineralöl (Siedebereich 255 bis > 377°C/Normaldruck) |

mischt und in einer Reibkugelmühle auf eine Feinheit von unter 5 Mikron vermahlt.

Beispiel D:

Ein emulgierbares Konzentrat wird erhalten aus

| | | |
|---|---|---|
| | 15 Gewichtsteilen | Wirkstoff |
| | 75 Gewichtsteilen | Cyclohexanon als Lösungsmittel |
| und | 10 Gewichtsteilen | oxäthyliertes Nonylphenol (10 AeO) als Emulgator. |

BIOLOGISCHE BEISPIELE

Beispiel I:

Weizenpflanzen wurden im 3-Blattstadium mit Konidien des Weizenmehltaus (Erysiphe graminis) stark inokuliert und in einem Gewächshaus bei 20°C und einer relativen Luftfeuchte von 90 - 95 % aufgestellt. 3 Tage nach Inokulation wurden die Pflanzen mit den in Tabelle I aufgeführten Verbindungen in den Wirkstoffkonzentrationen von 500, 250, 125, 60, 30 und 15 mg/Liter Spritzbrühe tropfnaß gespritzt. Als Vergleichsverbindung wurde Maneb eingesetzt. Nach einer Inkubationszeit von 10 Tagen wurden die Pflanzen auf Befall mit Weizenmehltau untersucht. Der Befallsgrad wurde ausgedrückt in % befallener Blattfläche bezogen auf unbehandelte, infizierte Kontrollpflanzen (= 100 % Befall). Das Ergebnis ist in der Tabelle I zusammengefaßt.

Tabelle I

| Verbindung gemäß Beispiel | mit Weizenmehltau befallene Blattfläche in % bei mg Wirkstoff/Liter Spritzbrühe 500 | 250 | 125 | 60 | 30 | 15 |
|---|---|---|---|---|---|---|
| 13 | 0 | 5 | 15 | 25 | | |
| 11 | 0 | 5 | 15 | 25 | | |
| 9 | 0 | 3-5 | 10 | 15 | | |
| 10 | 0 | 5 | 15 | 25 | | |
| 12 | 0-3 | 5 | 15 | 25 | | |
| 20 | 0-3 | 3 | 10 | 15 | | |
| 14 | 0-3 | 3 | 5 | 10 | | |
| 27 | 0 | 0 | 0 | 5 | 10-15 | |
| 1 | 0 | 0 | 3-5 | 10 | 15 | |
| 2 | 0 | 0 | 0 | 3-5 | 10 | |
| 35 | 0 | 0 | 0 | 0 | 5 | |
| 34 | 0 | 0 | 0 | 0 | 0-3 | 3-5 |
| 37 | 0 | 0 | 0 | 0 | 3 | 5 |
| 81 | 0 | 0 | 0 | 0 | 3 | 5 |
| 89 | 0 | 0 | 0 | 0 | 3 | 5 |
| 80 | 0 | 0 | 0 | 0 | 0 | 0 |
| 78 | 0 | 0 | 0 | 0 | 0 | 0 |
| 75 | 0 | 0 | 0 | 0-3 | 5 | 15 |
| 74 | 0 | 0 | 0 | 0 | 5 | 10 |
| 73 | 0 | 0 | 0-3 | 5-10 | 15 | 25 |
| 90 | 0 | 0 | 0 | 0 | 3 | 5 |
| Maneb *) | 5 | 10 | 15 | 25 | | |
| unbeh. infiz. Pflanzen | 100 | | | | | |

*) Maneb = Mangan(II)-(N,N'-äthylen-bis-(dithiocarbamat))

Beispiel II:

Gurkenpflanzen (sorte Delikateß) wurden im 2-Blattstadium mit einer Konidiensuspension von Gurkenmehltau (Erysiphe cichoracearum) stark inokuliert. Nach einer Antrocknungszeit der Sporensuspension von 30 Minuten wurden die Pflanzen in einem Gewächshaus bei 22°C und 90 % relativer Luftfeuchte aufgestellt. 3 Tage nach Infektion wurden die Pflanzen mit den in Tabelle II genannten Verbindungen und Wirkstoffkonzentrationen tropfnaß gespritzt. Als Vergleichsverbindung wurde Maneb eingesetzt. Nach 10 Tagen erfolgte die Bonitur. Der Befallsgrad wurde ausgedrückt in % befallener Blattfläche bezogen auf unbehandelte, infizierte Kontrollpflanzen (= 100 % Befall). Das Ergebnis ist in der Tabelle II zussammengefaßt.

Tabelle II

| Verbindung gemäß Beispiel | mit Gurkenmehltau befallene Blattfläche in % bei mg Wirkstoff/Liter Spritzbrühe | | | | | |
|---|---|---|---|---|---|---|
| | 500 | 250 | 125 | 60 | 15 | 8 |
| 13 | 0 | 3-5 | 10 | 15 | | |
| 11 | 0 | 3-5 | 5-10 | 15 | | |
| 9 | 0 | 3-5 | 10 | 15-25 | | |
| 10 | 0 | 3-5 | 15 | 25 | | |
| 12 | 0 | 3 | 3 | 5-10 | | |
| 20 | 0-3 | 5 | 10 | 15 | | |
| 14 | 0 | 0-3 | 5 | 10-15 | | |
| 25 | 0 | 0-3 | 5 | 10-15 | | |
| 27 | 0 | 0 | 0 | 0- 3 | 5-10 | |
| 1 | 0 | 0 | 0 | 0 | 0- 3 | |
| 2 | 0 | 0 | 0 | 0 | 3 | |
| 35 | 0 | 0 | 0 | 0 | 0 | 5 |
| 90 | 0 | 0 | 0 | 0 | 3 | |
| 81 | 0 | 0 | 0 | 0 | 3 | |
| 89 | 0 | 0 | 0 | 0 | 3 | |
| 80 | 0 | 0 | 0 | 0 | 0 | |
| 78 | 0 | 0 | 0 | 0 | 0 | |
| 75 | 0 | 0 | 0 | 0 | 0-3 | |
| 74 | 0 | 0 | 0 | 0 | 0-3 | |
| 73 | 0 | 0 | 0 | 0 | 0-3 | |
| Maneb *) | 15 | 25 | 35 | 60 | 100 | |
| unbeh. infiz. Pflanzen | 100 | | | | | |

*) vgl. Tabelle I

Beispiel III:

Gurkenpflanzen (Sorte Delikateß) wurden im 2-Blattstadium mit einer Konidiensuspension eines Benomyl-resistenten Gurkenmehltaustammes (Erysiphe cichoracearum) stark inokuliert. Nach einer Antrocknungszeit der Sporensuspension von 30 Minuten wurden die Pflanzen in einem Gewächshaus bei 22°C und 90 % relativer Luftfeuchte aufgestellt. 3 Tage nach Infektion wurden die Pflanzen mit den in Tabelle III genannten Verbindungen und Wirkstoffkonzentrationen tropfnaß gespritzt. Als Vergleichsverbindung wurde Maneb eingesetzt. Nach 10 Tagen erfolgte die Bonitur. Der Befallsgrad wurde ausgedrückt in % befallener Blattfläche bezogen auf unbehandelte, infizierte Kontrollpflanzen (= 100 % Befall). Das Ergebnis ist in der Tabelle III zusammengefaßt.

Tabelle III

| Verbindung gemäß Beispiel | mit Gurkenmehltau (Benomyl-resistenter Stamm) befallene Blattfläche in % bei mg Wirkstoff/ Liter Spritzbrühe | | | | | |
|---|---|---|---|---|---|---|
| | 500 | 250 | 125 | 60 | 15 | 7 |
| 13 | 0 | 3-5 | 10 | 15 | | |
| 11 | 0 | 5 | 5-10 | 15 | | |
| 9 | 0 | 5 | 10 | 15 | | |
| 10 | 0 | 3-5 | 15 | 25 | | |
| 12 | 0 | 0 | 0- 3 | 15-25 | | |
| 20 | 0 | 0-3 | 3- 5 | 15 | | |
| 14 | 0 | 3 | 5-10 | 15 | | |
| 25 | 0 | 0 | 3- 5 | 5-10 | | |
| 27 | 0 | 0 | 0 | 3 | 5 | 10 |
| 1 | 0 | 0 | 0 | 0 | 3-5 | 5 |
| 2 | 0 | 0 | 0 | 0 | 3-5 | 5 |
| 35 | 0 | 0 | 0 | 0 | 0 | 3 |
| 90 | 0 | 0 | 0 | 0 | 3-5 | |
| 81 | 0 | 0 | 0 | 0 | 3 | |

Tabelle III

| Verbindung gemäß Beispiel | mit Gurkenmehltau (Benomyl-resistenter Stamm) befallene Blattfläche in % bei mg Wirkstoff/ Liter Spritzbrühe | | | | | |
|---|---|---|---|---|---|---|
| | 500 | 250 | 125 | 60 | 15 | 7 |
| 89 | 0 | 0 | 0 | 0 | 3 | |
| 80 | 0 | 0 | 0 | 0 | 0 | |
| 78 | 0 | 0 | 0 | 0 | 0-3 | |
| 75 | 0 | 0 | 0 | 0 | 3-5 | |
| 74 | 0 | 0 | 0 | 0 | 3-5 | |
| 73 | 0 | 0 | 0 | 0 | 3 | |
| Maneb *) | 15 | 25 | 35 | 60 | 100 | 100 |
| unbeh. infiz. Pflanzen | 100 | | | | | |

*) vgl. Tabelle I

Beispiel IV:

Gerstenpflanzen wurden im 3-Blattstadium mit Konidien des Gerstenmehltaus (Erysiphe graminis sp. hordei) stark inokuliert und in einem Gewächshaus bei 20°C und einer relativen Luftfeuchte von 90 - 95 % aufgestellt. 3 Tage nach Inokulation wurden die Pflanzen mit den in Tabelle IV aufgeführten Verbindungen in den Wirkstoffkonzentrationen von 500, 250, 125, 60 und 30 mg/Liter Spritzbrühe tropfnaß gespritzt. Als Vergleichsverbindung wurde Maneb eingesetzt. Nach einer Inkubationszeit von 10 Tagen wurden die Pflanzen auf Befall mit Gerstenmehltau untersucht. Der Befallsgrad wurde ausgedrückt in % befallener Blattfläche bezogen auf unbehandelte, infizierte Kontrollpflanzen (= 100 % Befall). Das Ergebnis ist in der Tabelle IV zusammengefaßt.

Tabelle IV

| Verbindung gemäß Beispiel | mit Gerstenmehltau befallene Blattfläche in % bei mg Wirkstoff/Liter Spritzbrühe 500 | 250 | 125 | 60 | 30 |
|---|---|---|---|---|---|
| 13 | 0 | 5-10 | 15 | 25 | |
| 11 | 0 | 3 | 5-10 | 15 | |
| 9 | 0 | 3- 5 | 10 | 25 | |
| 10 | 0 | 3 | 10-15 | 25 | |
| 8 | 0-3 | 10 | 15 | 25 | |
| 12 | 0 | 5-10 | 15 | 25 | |
| 20 | 0-3 | 5-10 | 15 | 25 | |
| 14 | 0 | 5-10 | 15 | 25 | |
| 25 | 0-3 | 10-15 | 25 | 35 | |
| 27 | 0 | 0 | 0 | 3-5 | 10 |
| 1 | 0 | 0 | 0 | 0 | 3-5 |
| 2 | 0 | 0 | 0 | 0 | 3-5 |
| 35 | 0 | 0 | 3 | 3-5 | 5 |
| 34 | 0 | 0 | 0-3 | 5-10 | 15 |
| 37 | 0 | 0-3 | 3-5 | 10 | 15-25 |
| 90 | 0 | 0 | 0 | 0 | 3 |
| 81 | 0 | 0 | 0 | 0 | 0-3 |
| 89 | 0 | 0 | 0 | 0 | 3-5 |
| 80 | 0 | 0 | 0 | 0 | 0 |
| 78 | 0 | 0 | 0 | 0 | 0 |
| 75 | 0 | 0 | 0 | 0-3 | 5 |
| 74 | 0 | 0 | 0 | 0-3 | 3-5 |
| 73 | 0 | 0 | 0 | 0-3 | 3 |
| Maneb *) | 25 | 35 | 60 | 100 | 100 |
| unbeh.infiz. Pflanzen | 100 | | | | |

*) vgl. Tabelle 1

Beispiel V:

Apfelunterlagen der Sorte EM IX wurden im 4-Blattstadium mit einer Konidiensuspension von Apfelmehltau (Podosphaera leucotricha) stark infiziert. Anschließend kamen die Pflanzen für 16 Stunden in eine Klimakammer mit 20°C und einer relativen Luftfeuchte von ca. 100 %. Danach wurden die Pflanzen im Gewächshaus bei 22°C und einer relativen Luftfeuchte von 85 % aufgestellt. 3 Tage nach Infektion wurden die Pflanzen mit den in Tabelle V genannten Verbindungen und Wirkstoffkonzentrationen tropfnaß gespritzt. Als Vergleichsverbindung wurde Maneb eingesetzt. Nach 2 - 3 Wochen wurde der Mehltaubefall bonitiert und der Befallsgrad ausgedrückt in % befallener Blattfläche bezogen auf unbehandelte, infizierte Kontrollpflanzen (= 100 % Befall). Das Ergebnis ist in der Tabelle V zusammengefaßt.

Tabelle V

| Verbindung gemäß Beispiel | mit Apfelmehltau befallene Blattfläche in % bei mg Wirkstoff/Liter Spritzbrühe | | | |
|---|---|---|---|---|
| | 1000 | 500 | 250 | 125 |
| 12 | 0 | 0-3 | 5 | 15 |
| 14 | 0 | 0-3 | 5 | 25 |
| 27 | 0-3 | 3 | 5 | 15 |
| 1 | 0-3 | 3 | 5-10 | 15-25 |
| 2 | 0 | 0-3 | 3- 5 | 5 |
| 35 | 0 | 0-3 | 5 | 5 |
| Maneb *) | 25 | 35 | 60 | 100 |
| unbeh.infiz. Pflanzen | 100 | | | |

*) vgl. Tabelle I

Beispiel VI (Wuchshemmung)

In Schalenversuchen im Gewächshaus wurden junge Getreidepflanzen (Weizen, Gerste und Roggen) im 3-Blatt-Stadium mit der in Tabelle VI genannten Verbindung in den ange-

0007010

gebenen Wirkstoffkonzentrationen (kg/ha) tropfnaß gespritzt. Als Vergleichsverbindungen wurden 2-Chloräthyltrimethylammoniumchlorid, Maleinsäurehydrazid und Bernsteinsäure-2,2-dimethylhydrazid eingesetzt. Nachdem die unbehandelten Kontrollpflanzen eine Wuchshöhe von etwa 55 cm erreicht hatten, wurde bei allen Pflanzen der Zuwachs gemessen und die Wuchshemmung in % des Zuwachses der Kontrollpflanzen berechnet. Es wurde außerdem die phytotoxische Wirkung der Verbindungen beobachtet. Die Ergebnisse sind in der Tabelle VI zusammengefaßt. Bei der Angabe der Wuchshemmung bedeuten 100 % den Stillstand des Wachstums und 0 % ein Wachstum entsprechend dem der unbehandelten Kontrollpflanzen.

Tabelle VI

Wuchshemmung bei Getreide

| Verbindung gemäß Beispiel | Anwendungskonzentration (kg/ha) | Wuchshemmung in % Weizen | Gerste | Roggen | Phytotoxische Wirkung |
|---|---|---|---|---|---|
| 127 | 2,5<br>1,25 | 33<br>30 | 29<br>28 | 26<br>10 | keine Schäden |
| Vergleich (2-Chloräthyl)-trimethylammoniumchlorid) | 2,5<br>1,25 | 27<br>23 | 8<br>0 | 10<br>0 | keine Schäden |
| Vergleich Maleinsäure hydrazid | 2,5<br>1,25 | 37<br>20 | 35<br>20 | 38<br>29 | starke Schäden |
| Vergleich Bernsteinsäure-2,2-dimethylhydrazid | 2,5<br>1,25 | 0<br>0 | 0<br>0 | 0<br>0 | keine Schäden |

Patentansprüche:

1. Verbindungen der allgemeinen Formel I,

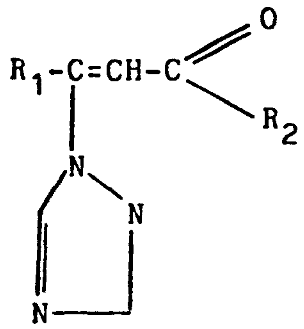

(I)

in der

$R_1$ Methyl oder Phenyl, das gegebenenfalls durch Halogenatome, $(C_1-C_4)$-Alkylreste oder $(C_1-C_4)$-Alkoxyreste substituiert ist, und

$R_2$ eine $R_3O$-Gruppe, in der $R_3$ Alkyl mit bis zu 12 C-Atomen, das gegebenenfalls zusätzlich durch Halogen, $(C_1-C_4)$-Alkoxyreste oder Alkoxycarbonylreste mit bis zu 8 C-Atomen substituiert ist, Alkinyl mit bis zu 3 C-Atomen, Cycloalkyl, mit bis zu 6 C-Atomen, das gegebenenfalls zusätzlich durch $(C_1-C_4)$-Alkylreste substituiert ist, Phenyl, das gegebenenfalls durch Halogenatome und/oder die $F_3C$-Gruppe substituiert ist, oder

$R_2$ eine 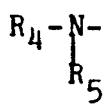-Gruppe,

in der $R_4$ Wasserstoff oder $(C_1-C_4)$-Alkyl, das gegebenenfalls durch Alkoxycarbonyl mit bis zu 8 C-Atomen substituiert ist, und $R_5$ $(C_1-C_4)$-Alkyl oder Phenyl, das gegebenenfalls durch Halogenatome, $F_3C$-Reste, $(C_1-C_4)$-Alkyl- oder $(C_1-C_4)$-Alkoxyreste oder durch Phenoxy oder Halogenphenoxy substituiert ist, bedeutet.

2. Verfahren zur Herstellung von Verbindungen der Formel I gemäß Anspruch 1, dadurch gekennzeichnet, daß man Verbindungen der allgemeinen Formel II,

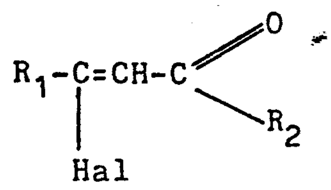

(II)

in der $R_1$ und $R_2$ die Bedeutungen wie in Formel I haben und Hal Chlor oder Brom bedeutet, bei höheren Temperaturen mit 1,2,4-Triazol in Gegenwart eines Halogenwasserstoff bindenden Mittels umsetzt.

3. Verfahren nach Anspruch 2, dadurch gekennzeichnet, daß man die Umsetzung in einem organischen Lösungsmittel durchführt.

4. Verfahren nach Ansprüchen 2 und 3, dadurch gekennzeichnet, daß man zum Abfangen der bei der Umsetzung gebildeten Halogenwasserstoffsäure einen mindestens molaren Überschuß an Triazol oder mindestens äquivalente Mengen anorganischer oder tertiärer organischer Basen oder deren Gemische einsetzt.

5. Schädlingsbekämpfungsmittel, gekennzeichnet durch einen Gehalt an einer Verbindung der Formel I gemäß Anspruch 1 als Wirkstoff.

6. Schädlingsbekämpfungsmittel, gekennzeichnet durch einen Gehalt von 2 bis 95 Gew.-% an einer Verbindung der Formel I gemäß Anspruch 1 als Wirkstoff sowie üblichen Formulierungshilfsmitteln.

7. Verwendung von Verbindungen der Formel I gemäß Ansprüchen 1, 5 und 6 zur Schädlingsbekämpfung im Pflanzenschutz.

8. Verwendung von Verbindungen der Formel I gemäß Ansprüchen 1, 5 und 6 als Pflanzenwuchsregulatoren.

9. Verwendung von Verbindngen der Formel I gemäß Anspruch 8 zur Wachstumshemmung in Getreide.

Patentansprüche für Österreich

1. Verfahren zur Herstellung von Verbindungen der allgemeinen Formel I,

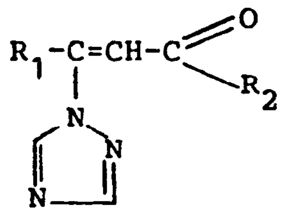

(I)

in der

$R_1$ Methyl oder Phenyl, das gegebenenfalls durch Halogenatome, $(C_1-C_4)$-Alkylreste oder $(C_1-C_4)$-Alkoxyreste substituiert ist, und

$R_2$ eine $R_3O$-Gruppe, in der $R_3$ Alkyl mit bis zu 12 C-Atomen, das gegebenenfalls zusätzlich durch Halogen, $(C_1-C_4)$-Alkoxyreste oder Alkoxycarbonylreste mit bis zu 8 C-Atomen substituiert ist, Alkinyl mit bis zu 3 C-Atomen, Cycloalkyl mit bis zu 6 C-Atomen, das gegebenenfalls zusätzlich durch $(C_1-C_4)$-Alkylreste substituiert ist, Phenyl, das gegebenenfalls durch Halogenatome und/oder die $F_3C$-Gruppe substituiert ist, oder

$R_2$ eine $R_4-\underset{R_5}{\underset{|}{N}}$-Gruppe,

in der $R_4$ Wasserstoff oder $(C_1-C_4)$-Alkyl, das gegebenenfalls durch Alkoxycarbonyl mit bis zu 8 C-Atomen substituiert ist, und $R_5$ $(C_1-C_4)$-Alkyl oder Phenyl, das gegebenenfalls durch Halogenatome, $F_3C$-Reste, $(C_1-C_4)$-Alkyl- oder $(C_1-C_4)$-Alkoxyreste oder durch Phenoxy oder Halogenphenoxy substituiert ist, bedeutet, dadurch gekennzeichnet, daß man Verbindungen der allgemeinen Formel II,

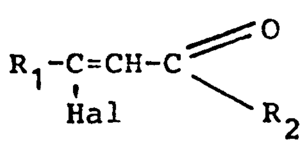

(II)

in der $R_1$ und $R_2$ die Bedeutungen wie in Formel I haben und Hal Chlor oder Brom bedeutet, bei höheren Temperaturen mit 1,2,4-Triazol in Gegenwart eines Halogenwasserstoff bindenden Mittels umsetzt.

2. Verfahren nach Anspruch 1, dadurch gekennzeichnet, daß man die Umsetzung in einem organischen Lösungsmittel durchführt.

3. Verfahren nach Ansprüchen 1 und 2, dadurch gekennzeichnet, daß man zum Abfangen der bei der Umsetzung gebildeten Halogenwasserstoffsäure einen mindestens molaren Überschuß an Triazol oder mindestens äquivalente Mengen anorganischer oder tertiärer organischer Basen oder deren Gemische einsetzt.

4. Schädlingsbekämpfungsmittel, gekennzeichnet durch einen Gehalt an einer Verbindung der Formel I gemäß Anspruch 1 als Wirkstoff.

5. Schädlingsbekämpfungsmittel, gekennzeichnet durch einen Gehalt von 2 bis 95 Gew.-% an einer Verbindung der Formel I gemäß Anspruch 1 als Wirkstoff sowie üblichen Formulierungshilfsmitteln.

6. Verwendung von Verbindungen der Formel I gemäß Ansprüchen 1, 4 und 5 zur Schädlingsbekämpfung im Pflanzenschutz.

7. Verwendung von Verbindungen der Formel I gemäß Ansprüchen 1, 4 und 5 als Pflanzenwuchsregulatoren.

8. Verwendung von Verbindungen der Formel I gemäß Anspruch 7 zur Wachstumshemmung in Getreide.

0007010

Europäisches Patentamt

# EUROPÄISCHER RECHERCHENBERICHT

Nummer der Anmeldung

EP 79 10 1917

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| AP | US - A - 4 113 465 (SHEPHARD)<br>Ansprüche *<br>-- | 5-7 |
| A | DE - A - 2 407 143 (BAYER)<br>* Seiten 1,2 *<br>-- | 8,9 |
| A | GB - A - 1 511 956 (J.C.J.)<br>* Seiten 1-3 *<br>-- | 1-9 |
| A | DE - A - 2 638 470 (J.C.J.)<br>* Ansprüche 1-5 *<br>-- | 1-4 |
| A | DE - A - 2 634 511 (BASF)<br>* Ansprüche *<br>---- | 1-4 |

KLASSIFIKATION DER ANMELDUNG (Int.Cl.³)

C 07 D 249/08
A 01 N 43/64

RECHERCHIERTE SACHGEBIETE (Int. Cl.³)

C 07 D 249/08
A 01 N 5/00
9/22

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

Der vorliegende Recherchenbericht wurde für alle Patentansprüche erstellt

| Recherchenort | Abschlußdatum der Recherche | Prüfer |
|---|---|---|
| Wien | 10.08.1979 | HAMMER |